# EUROPEAN PATENT SPECIFICATION

(11) **EP 3 407 834 B1**
(45) Date of publication and mention of the grant of the patent: **24.08.2022**
(21) Application number: 17701977.5
(22) Date of filing: 17.01.2017
(51) Int. Cl.: A61F 2/24

(54) **SYSTEMS FOR REPOSITIONING A FULLY DEPLOYED VALVE ASSEMBLY**
SYSTEME ZUR NEUPOSITIONIERUNG EINER VOLLSTÄNDIG AUSGEFAHRENEN VENTILANORDNUNG
SYSTÈMES POUR REPOSITIONNER UN ENSEMBLE VALVULE ENTIÈREMENT DÉPLOYÉ

(30) Priority: 27.01.2016 US 201615008019
(43) Date of publication of application: 05.12.2018
(73) Proprietor: Medtronic Inc., Minneapolis, MN 55432 (US)
(72) Inventor: CUNNINGHAM, Kieran, Santa Rosa California 95403 (US); LALLY, Marian, Santa Rosa California 95403 (US)
(74) Representative: Zimmermann & Partner Patentanwälte mbB
(86) International application number: PCT/US2017/013807
(87) International publication number: WO 2017/132008

(56) References cited:
- US-A1- 2007 100 427
- US-A1- 2008 039 934
- US-A1- 2011 264 198
- US-A1- 2014 249 564

## Description

### FIELD OF THE INVENTION

The present disclosure relates to devices, systems, and methods for repositioning a fully deployed valve assembly. The methods described herein do not form part of the invention.

### BACKGROUND

Heart valves are sometimes damaged by disease or by aging, resulting in problems with the proper functioning of the valve. Heart valve replacement has become a routine surgical procedure for patients suffering from valve dysfunctions. Traditional open surgery inflicts significant patient trauma and discomfort, requires extensive recuperation times, and may result in life-threatening complications.

To address these concerns, minimally invasive techniques, such as transcatheter valve implantation techniques, have been developed to deliver and deploy valve prostheses. In such methods, the valve prosthesis or valve assembly generally includes a frame and a prosthetic valve, and is radially compressed for delivery in a catheter and then advanced to the location of a native valve, where the valve assembly is deployed by radial expansion. The catheter may be advanced, for example through an opening in the native vasculature remote from the native valve, such as the femoral artery, and advanced through the vasculature to the native valve. In other techniques, the catheter is advanced through an opening in the heart to the location of the native valve, such as transapical or transatrially, or through an opening in the ascending aorta.

In some patients, the valve assembly may not perform as desired following implantation. For example, due to the position of the valve assembly, the valve assembly may not properly seal with the native valve and/or walls surrounding the native valve. This may result in paravalvular leakage (PVL), and other post surgical complications. Further, the valve assembly may not function properly due to the position of the valve assembly at the native valve. However, once a valve assembly is fully deployed and released from the delivery device, there is no easy way to reposition to the valve assembly to a new location. US 2007/0100427 A1 describes a device for treating a blood vessel and associated treatment kit. US 2008/0039934 A1 describes an interchangeable prosthetic valve. US 2014/0249564 A1 describes methods of repositioning a transcatheter heart valve after full deployment.

Accordingly, there is a need for a valve assembly, system and method of repositioning a fully deployed valve assembly.

### SUMMARY OF INVENTION

Embodiments hereof relate to a valve assembly including a frame, a prosthetic valve, and a repositioning wire. The frame defines a central passage. The prosthetic valve is coupled to the frame and disposed in the central passage of the frame. The repositioning wire is coupled to the frame. The repositioning wire is configured such that with the valve assembly in a radially expanded fully deployed configuration, pulling the repositioning wire radially compresses the valve assembly from the radially expanded fully deployed configuration to a radially compressed repositioning configuration, as defined by claim 1.

Embodiments hereof also relate to a valve assembly repositioning system for repositioning a valve assembly that is in a radially expanded fully deployed configuration. The valve assembly repositioning system includes the valve assembly and a snare device. The valve assembly includes a frame that defines a central passage, a prosthetic valve coupled to the frame, and a repositioning wire coupled to the frame. The snare device is configured to snare and pull the repositioning wire to radially compress the valve assembly from the radially expanded fully deployed configuration to a radially compressed repositioning configuration. The snare device is also configured to move the valve assembly when the valve assembly is in the radially compressed repositioning configuration.

Examples that do not form part of the invention hereof also relate to a method of repositioning a valve assembly having a frame, a prosthetic valve coupled to the frame, and a repositioning wire coupled to the frame. The method includes advancing a snare device to a location of the valve assembly with the valve assembly in a radially expanded fully deployed configuration at a first location adjacent a native valve. The snare device snares the repositioning wire. The snare device is manipulated such that the repositioning wire is pulled to radially compress the valve assembly from the radially expanded fully deployed configuration to a radially compressed repositioning configuration. The valve assembly is moved from the first location to a second location adjacent the native valve by manipulation of the snare device. The repositioning wire is released from the snare device and the valve assembly radially expands from the radially compressed repositioning configuration back to the radially expanded fully deployed configuration.

### BRIEF DESCRIPTION OF DRAWINGS

FIG. 1A is a side perspective schematic illustration of a valve assembly according to an embodiment hereof with the valve assembly in a radially expanded fully deployed configuration.
FIG. 1B an end view schematic illustration of the valve assembly of FIG. 1A.
FIG. 2A is a side view schematic illustration of the valve assembly of FIG. 1A in a radially compressed repositioning configuration.
FIG. 2B is an end view schematic illustration of the valve assembly of FIG. 1A in a radially compressed repositioning configuration.
FIG. 3 is a side perspective schematic illustration of a valve assembly repositioning system according to an embodiment hereof, with the valve assembly of FIG. 1A.
FIGS. 4A-4D are a series of side illustrations of a snare device grasping and pulling a repositioning wire.
FIG. 5A is a side perspective schematic illustration of a valve assembly according to another embodiment hereof, wherein the repositioning wire includes a lasso.
FIG. 5B is an end view perspective schematic illustration of the valve assembly of FIG. 5A.
FIG. 6 is a side perspective schematic illustration of a valve assembly repositioning system according to an embodiment hereof, with the valve assembly of FIG. 5A.
FIGS. 7A-7E are a series of close up side illustrations of snare device snaring and pulling the lasso of the repositioning wire.
FIG. 8A is a side perspective schematic illustration of the valve assembly of FIG. 5A, wherein the valve assembly includes a plurality of repositioning wires with lassos.
FIG. 8B is an end view perspective schematic illustration of the valve assembly of FIG. 8A.
FIG. 9 is a side perspective schematic illustration of a valve assembly repositioning system according to an embodiment hereof, with the valve assembly of FIG. 8.
FIGS. 10A-10D are a series of side schematic illustrations of a snare device snaring and pulling the lassos of the repositioning wires of the valve assembly of FIG. 8.
FIGS. 11-16 are schematic illustrations of an example not forming part of the invention of a method of repositioning a fully deployed valve assembly.

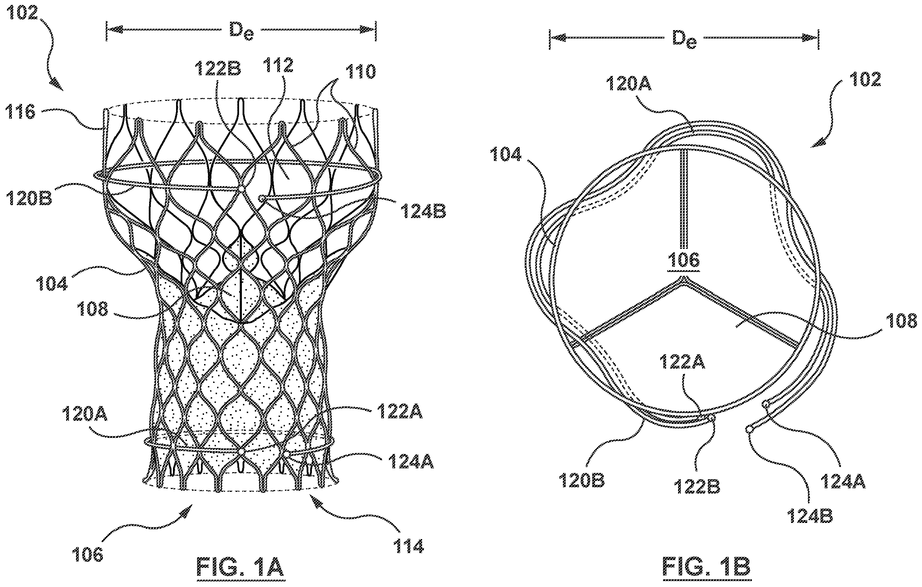

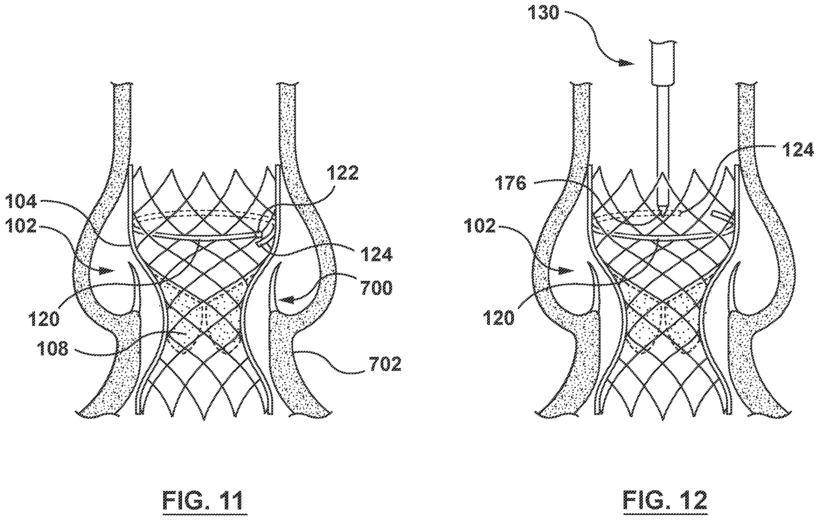

FIG. 11 is a schematic illustration of the valve assembly of FIG. 1A in a radially expanded fully deployed configuration and disposed at a first location adjacent a native valve.
FIG. 12 is a schematic illustration a step in the method, not forming part of the invention, of repositioning the valve assembly, wherein the snare device has grasped the repositioning wire.

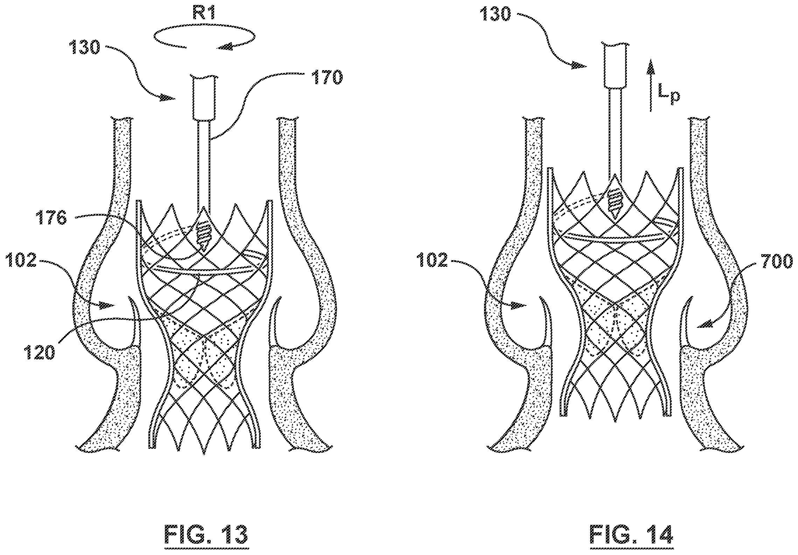

FIG. 13 is a schematic illustration of a step in the method, not forming part of the invention, of repositioning the valve assembly, wherein the snare device is pulling the repositioning wire and the valve assembly is collapsing to the radially collapsed repositioning configuration.
FIG. 14 is a schematic illustration of a step in the method, not forming part of the invention, of repositioning the valve assembly, wherein the valve assembly is in the radially collapsed repositioning configuration and is being moved to a second location adjacent the native valve.

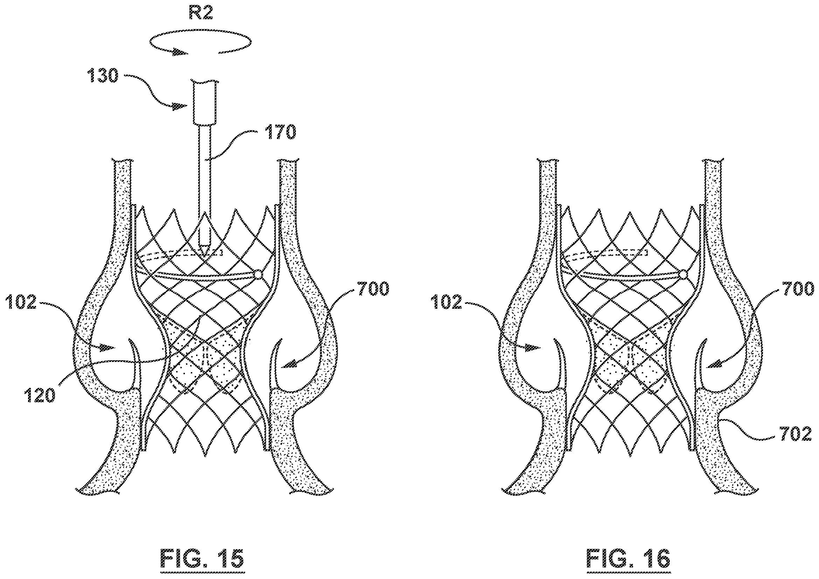

FIG. 15 is a schematic illustration of a step in the method, not forming part of the invention, of repositioning the valve assembly, wherein the snare device is releasing the repositioning wire and the valve assembly is expanding to the radially expanded fully deployed configuration at the second location.
FIG. 16 is a schematic illustration of a step in the method, not forming part of the invention, of repositioning the valve assembly, wherein the snare device has released the valve assembly such that the valve assembly is in the radially expanded fully deployed configuration at the second location.

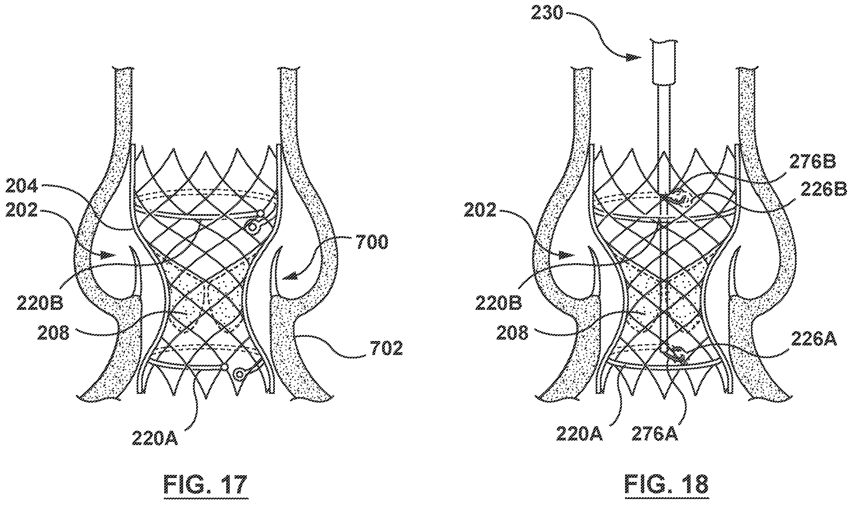

FIGS. 17-22 are schematic illustrations of another example not forming part of the invention of a method of repositioning a fully deployed valve assembly.

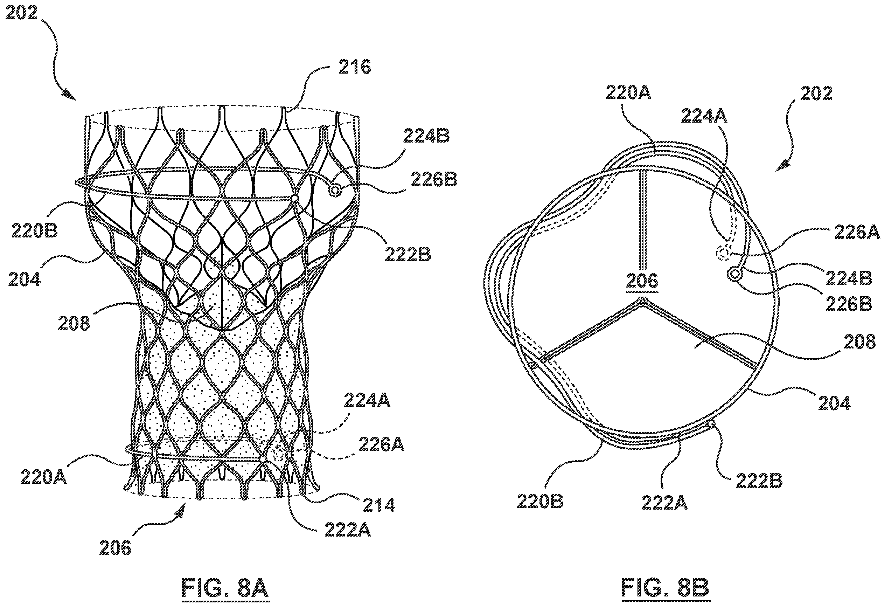

FIG. 17 is a schematic illustration of the valve assembly of FIG. 8A in a radially expanded fully deployed configuration and disposed at a first location adjacent a native valve.
FIG. 18 is a schematic illustration of a step in the method, not forming part of the invention, of repositioning the valve assembly, wherein the snare device has snared the lassos of the repositioning wires.

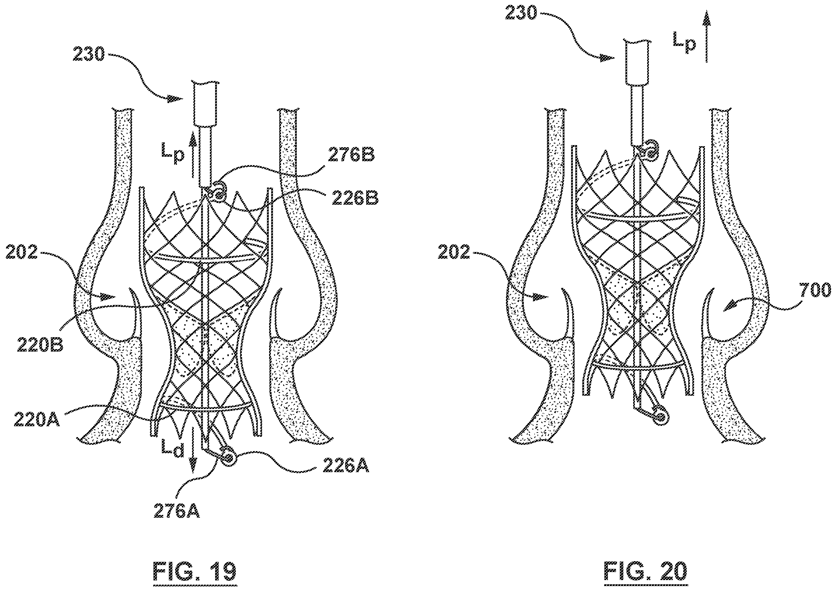

FIG. 19 is a schematic illustration of a step in the method, not forming part of the invention, of repositioning the valve assembly, wherein the snare device is pulling the repositioning wires to collapse the valve assembly to the radially collapsed repositioning configuration.
FIG. 20 is a schematic illustration of a step in the method, not forming part of the invention, of repositioning the valve assembly, wherein the valve assembly is in the radially collapsed repositioning configuration and is being moved to a second location adjacent the native valve.

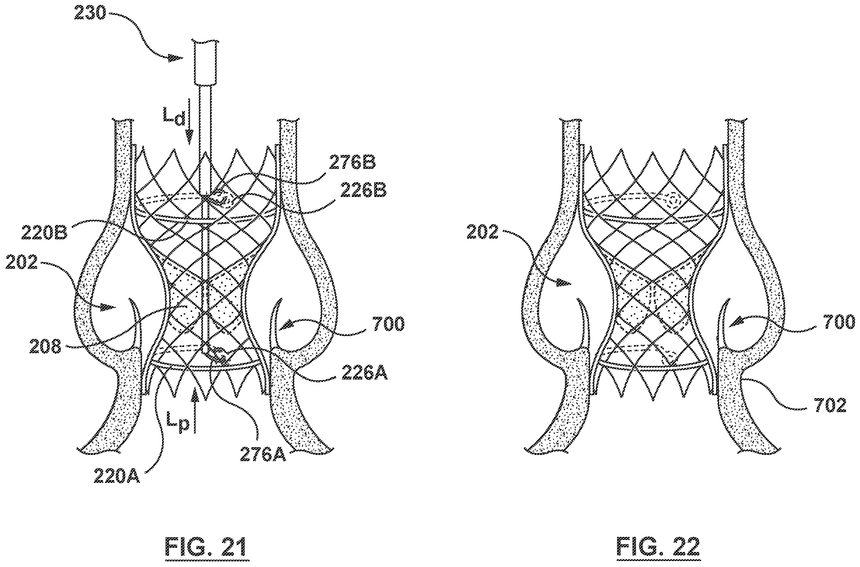

FIG. 21 is a schematic illustration of a step in the method, not forming part of the invention, of repositioning the valve assembly, wherein the snare device is releasing the repositioning wire and the valve assembly is expanding to the radially expanded fully deployed configuration at the second location.
FIG. 22 is a schematic illustration of a step in the method, not forming part of the invention, of repositioning the valve assembly, wherein the snare device has released the valve assembly such that the valve assembly is in the radially expanded fully deployed configuration at the second location.

### DETAILED DESCRIPTION

Specific embodiments of the present invention are now described with reference to the figures, wherein like reference numbers indicate identical or functionally similar elements. The terms "distal" and "proximal", when used in the following description to refer to a catheter or delivery device, are with respect to a position or direction relative to the treating clinician. Thus, "distal" and "distally" refer to positions distant from, or in a direction away from, the clinician and "proximal" and "proximally" refer to positions near, or in a direction toward, the clinician. When the terms "distal" and "proximal" are used in the following description to refer to a device implanted into a native artery, such as a valve assembly, they are used with reference to the direction of blood flow from the heart. Thus "distal" and "distally" refer to positions in a downstream direction with respect to the direction of blood flow and "proximal" and "proximally" refer to positions in an upstream direction with respect to the direction of blood flow.

The following detailed description is merely exemplary in nature and is not intended to limit the invention or the application and uses of the invention. Although the description of the invention is in the context of a transcatheter aortic valve repositioning system, the invention may also be used in other body passageways where it is deemed useful. Furthermore, there is no intention to be bound by any expressed or implied theory presented in the preceding technical field, background, brief summary, or the following detailed description.

As used herein the terms "fully deployed", "fully deployed configuration", and "radially expanded fully deployed configuration" mean that the device, such as a valve assembly or frame, that is described using these terms has been deployed at a site within the body, has been radially expanded (such as by balloon expansion or selfexpansion), and has been released from the delivery device. Thus, for example, a valve assembly wherein a portion of the valve assembly has been radially expanded but a portion of the valve assembly is either not radially expanded or is still attached to the delivery device, is not considered "fully deployed", in a "fully deployed configuration", or in a "radially expanded fully deployed configuration".

The valve assembly of the present invention includes a frame, a prosthetic valve, and at least one repositioning wire. The valve assembly has a radially expanded fully deployed configuration that is collapsible to a radially compressed repositioning configuration for repositioning the valve assembly after the valve assembly has been fully deployed adjacent a native valve. The valve assembly also has a radially compressed delivery configuration (not shown), which is a smaller diameter than the radially compressed repositioning configuration.

The frame of the valve assembly is a generally tubular configuration having a proximal end, a distal end, and a lumen therebetween. The frame is a stent structure as is known in the art, as described in more detail below. The frame may be self expanding or may be balloon expandable. The frame may comprise a number of strut or wire portions arranged relative to each other to provide a desired compressibility, strength, and leaflet attachment zone(s). The frame is a generally tubular support structure, and leaflets are secured to the frame to provide a stented prosthetic valve.

The prosthetic valve of the valve assembly is attached to the frame. The prosthetic valve may also include a skirt affixed to the frame. The prosthetic valve may include a plurality of prosthetic valve leaflets, which may be attached along their bases to the skirt, for example, using sutures or a suitable biocompatible adhesive, or may be attached to the skirt or frame in other ways known to those skilled in the art. Adjoining pairs of leaflets may be attached to one another at their lateral ends to form commissures with free edges of the leaflets forming coaptation edges that meet in an area of coaptation. The prosthetic valve leaflets may be formed from a variety of materials, such as autologous tissue, xenograph material, or synthetics as are known in the art. The leaflets may be provided as a homogenous, biological valve structure, such as a porcine, bovine, or equine valve. Alternatively, the leaflets can be provided independent of one another (e.g., bovine or equine pericardial leaflets) and subsequently assembled to the support structure of the the frame. In another alternative, the frame and leaflets may be fabricated at the same time, such as may be accomplished using high strength nano-manufactured NiTi films of the type produced at Advanced Bio Prosthetic Surfaces Ltd. (ABPS) of San Antonio, Tex., for example.

The frame and prosthetic valve of the valve assembly may be similar to the Medtronic CoreValve^{®} transcatheter aortic valve replacement valve prosthesis and as described in U.S. Patent Application Publication No. 2011/0172765 to Nguyen al. However, those skilled in the art would recognize that any suitable valve prosthesis may be used in the present embodiment as the frame and prosthetic valve of the valve assembly. For example, and not by way of limitation, the combination of a frame and prosthetic valve of the valve assembly may assume a variety of other configurations that differ from those shown and described, including any known prosthetic heart valve design. In various embodiments, the frame and the prosthetic valve may utilize certain features of known expandable prosthetic heart valve configurations, whether balloon expandable, selfexpanding, or unfurling (as described, for example, in U.S. Pat. Nos. 3,671,979; 4,056,854; 4,994,077; 5,332,402; 5,370,685; 5,397,351; 5,554,185; 5,855,601; and 6,168,614; U.S. Patent Application Publication No. 2004/0034411; Bonhoeffer P., et al., "Percutaneous Insertion of the Pulmonary Valve", Pediatric Cardiology, 2002; 39:1664-1669; Anderson H R, et al., "Transluminal Implantation of Artificial Heart Valves", EUR Heart J., 1992; 13:704-708; Anderson, J. R., et al., "Transluminal Catheter Implantation of New Expandable Artificial Cardiac Valve", EUR Heart J., 1990, 11: (Suppl) 224a; Hilbert S. L., "Evaluation of Explanted Polyurethane Trileaflet Cardiac Valve Prosthesis", J Thorac Cardiovascular Surgery, 1989; 94:419-29; Block P C, "Clinical and Hemodynamic Follow-Up After Percutaneous Aortic Valvuloplasty in the Elderly", The American Journal of Cardiology, Vol. 62, Oct. 1, 1998; Boudjemline, Y., "Steps Toward Percutaneous Aortic Valve Replacement", Circulation, 2002; 105:775-558; Bonhoeffer, P., "Transcatheter Implantation of a Bovine Valve in Pulmonary Position, a Lamb Study", Circulation, 2000: 102:813-816; Boudjemline, Y., "Percutaneous Implantation of a Valve in the Descending Aorta In Lambs", EUR Heart J, 2002; 23:1045-1049; Kulkinski, D., "Future Horizons in Surgical Aortic Valve Replacement: Lessons Learned During the Early Stages of Developing a Transluminal Implantation Technique", ASAIO J, 2004;).

The valve assembly of the present invention adds at least one repositioning wire. The term "wire" as used herein means an elongated element or filament or group of elongated elements or filaments and is not limited to a particular crosssectional shape or material, unless so specified. The repositioning wire of the present disclosure includes a first end coupled to the frame. The repositioning wire extends from the first end around a circumference of the frame to a second end disposed opposite the first end. The first end of the repositioning wire may be coupled to the frame of the valve assembly by methods such as, but not limited to laser or ultrasonic welding, adhesives, tying, or other methods suitable for the purposes disclosed herein. The repositioning wire wraps around at least a portion of the circumference of the valve assembly. In an embodiment, with the valve assembly is in the radially expanded fully deployed configuration, the repositioning wire wraps around at least 75% of the circumference of the frame at the location of the repositioning wire. The repositioning wire may be woven through the open spaces of the frame of the valve assembly, above some frame members and below others. The repositioning wire is configured such that when the second end of the repositioning wire is pulled, the valve assembly is compressed from the radially expanded fully deployed configuration to the radially compressed repositioning configuration. The repositioning wire may be constructed of materials such as, but not limited to stainless steel, Nitinol, nylon, polybutester, polypropylene, silk, and polyester or other materials suitable for the purposes described herein.

The present disclosure also discloses a valve assembly repositioning system, which includes a valve assembly, as described above, and a snare device. The snare device is an elongate member configured to snare and to pull the repositioning wire of the valve assembly such that the valve assembly is compressed from the radially expanded fully deployed configuration to the radially compressed repositioning configuration. The snare device is also configured to move the valve assembly from a first location adjacent a native valve to a second location adjacent a native valve when the valve assembly is in the radially compressed repositioning configuration.

With the above understanding in mind, an embodiment of a valve assembly 102 according to the present disclosure is shown in FIGS. 1A-2B. FIG. 1A illustrates valve assembly 102 in a radially expanded fully deployed configuration. Valve assembly 102 includes a frame 104, a prosthetic valve 108, and a repositioning wire 120, as described above.

Frame 104 is of a generally tubular configuration including a first end 104 and a second end 106, and defines a central passage 106 therethrough. Frame 104 is a support structure that comprises a number of wire members 110 arranged relative to each other to create open spaces 112. Prosthetic valve 108 is coupled to frame 104 and disposed within central passage 106 of frame 104.

Valve assembly 102 further includes a first repositioning wire 120A and a second repositioning wire 120B. In the embodiment shown, first repositioning wire 120A is disposed adjacent first end 114 (inflow end) of frame 104 and second repositioning wire 120B is disposed adjacent second end 116 (outflow end) of frame 104. Although FIGS. 1A-2B show two repositioning wires, more or fewer repositioning wires may be utilized. For example, and not by way of limitation, a single reposition wire may be utilized adjacent the first end, second end, or middle portion of frame 104. Alternatively, and also not by way of limitation, a third repositioning wire may be added between the first and second repositioning wires. As explained above, first repositioning wire 120A includes a first end 122A coupled to frame 104. First repositioning wire 120A wraps around a circumference of frame 104 to a second end 124A, as shown in FIG. 1A. Similarly, second repositioning wire 120B includes a first end 122B coupled to frame 104. Second repositioning wire 120B wraps around a circumference of frame 104 to a second end 124B, as shown in FIG. 1A. In an embodiment, with valve assembly 102 in the radially expanded fully deployed configuration, each of first and second repositioning wires 120A, 120B wraps around the circumference of frame 104 at least 75% of the circumference of frame 104 at the location of each respective repositioning wire. As previously described, each repositioning wire 120A, 120B may be woven through open spaces 112 of frame 104, above some frame members 110 and below others.

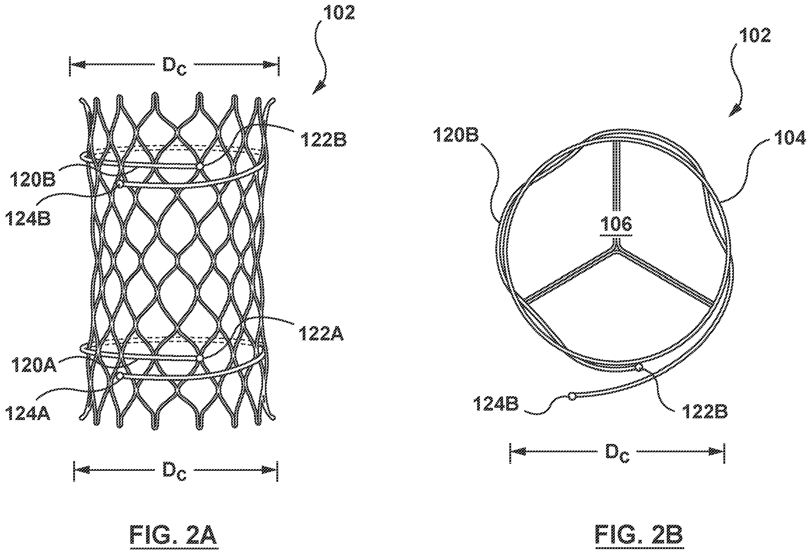

Each repositioning wire 120A, 120B is configured such that when respective second end 124A, 124B of each repositioning wire 120A, 120B is pulled, frame 104 of valve assembly 102 is compressed from the radially expanded fully deployed configuration to a radially compressed repositioning configuration, thereby also compressing valve assembly 102 from the radially expanded fully deployed configuration to the radially compressed repositioning configuration. As shown in FIGS. 1A-2B, valve assembly 102 has an outer diameter Dₑ when in the radially expanded fully deployed configuration, as shown in FIGS. 1A-1B which is greater than an outer diameter D_{c} when in the radially compressed repositioning configuration, as shown in FIGS. 2A-2B. Outer diameter D_{c} may be in the range of 40% to 80% of outer diameter Dₑ.

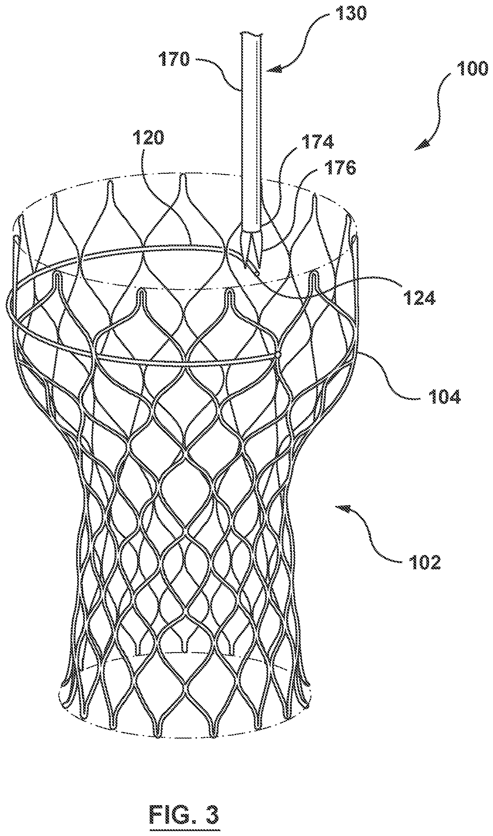

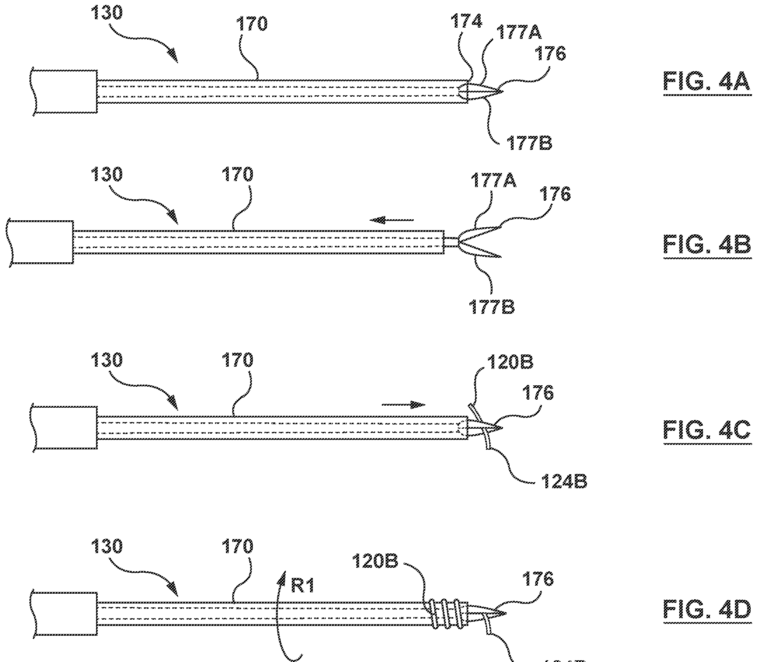

FIG. 3 shows an embodiment of a valve assembly repositioning system 100 including valve assembly 102 and a snare device 130. Snare device 130, shown in more detail in FIGS. 4A-4D, is an elongated device including a shaft 170 configured for delivery through the vasculature, and a clasping mechanism 176 disposed at a distal end 174 of shaft 170 of snare device 130. More particularly, clasping mechanism 176 is user actuated from a location at a proximal end (not shown) of snare device 130. Clasping mechanism 176 is configured to grasp and hold second end 124B of repositioning wire 120B, as shown in FIG. 3. Although snare device 130 in FIG. 3 is shown grasping repositioning wire 120B, snare device 130 could instead grasp repositioning wire 120A, or snare device 130 may include a plurality of clasping mechanisms, one for each repositioning wire, or a plurality of snare devices 130 could be used, one for each repositioning wire. Snare device 130 is further configured to pull repositioning wire 120B such that valve assembly 102 is compressed from the radially expanded fully deployed configuration to the radially compressed repositioning configuration. In the embodiment shown in FIGS. 3and 4A-4D, repositioning wire 120B is pulled by rotating snare device 130 in a direction R1 such that repositioning wire 120B wraps circumferentially around shaft 170 of snare device 130, as shown in FIGS. 4A-4D. To release repositioning wire 120B, snare device 130 is rotated in a direction (not shown) opposite direction R1.

Snare device 130 is also configured to move valve assembly 102 longitudinally within the native vessel or valve when valve assembly 102 is in the radially compressed repositioning configuration. To move valve assembly 102 distally or proximally, the user pushes or pulls snare device 130, respectively. Stated another way, when snare device 130 has grasped and pulled repositioning wire 120 and valve assembly 102 is in the radially compressed repositioning configuration, moving snare device 130 distally moves valve assembly 102 distally (i.e., away from the clinician), and moving snare device 130 proximally moves valve assembly 102 proximally (i.e., towards the clinician).

Clasping mechanism 176 shown in FIGS. 3-4D is shown including a pair of jaws 177A, 177B. However, any clasping mechanism suitable to grasp and hold a repositioning wire may be utilized. In one embodiment, jaws 177A, 177B are displaceable towards and away from one another and are formed from a resilient material. In an embodiment, jaws 177A, 177B are biased into a normally open configuration, as shown in FIG. 4B. For delivery to the location of valve assembly 102, shaft 170 is extended at least partially over clasping mechanism 176 to maintain jaws 177A, 177B in a closed configuration. When it is desired to open jaws 177A, 177B, shaft 170 is retracted proximally to expose jaws 177A, 177B such that their natural bias opens jaws 177A, 177B, as shown in FIG. 4B. With the repositioning wire 120B disposed between jaws 177A, 177B, shaft 170 is moved distally to force jaws 177A, 177B together, as shown in FIG. 4C. Snare device 130 may then be rotated in direction R1, as shown in FIG. 4D, to pull the repositioning wire by wrapping it around shaft 170, as explained above. Other clasping mechanisms may be utilized. For example, and not by way of limitation, the jaws may be opening and closed by a mechanical linkage extending proximally to a handle which is operated by the user. Other clasping mechanisms which do not necessarily include two jaws, may also be utilized.

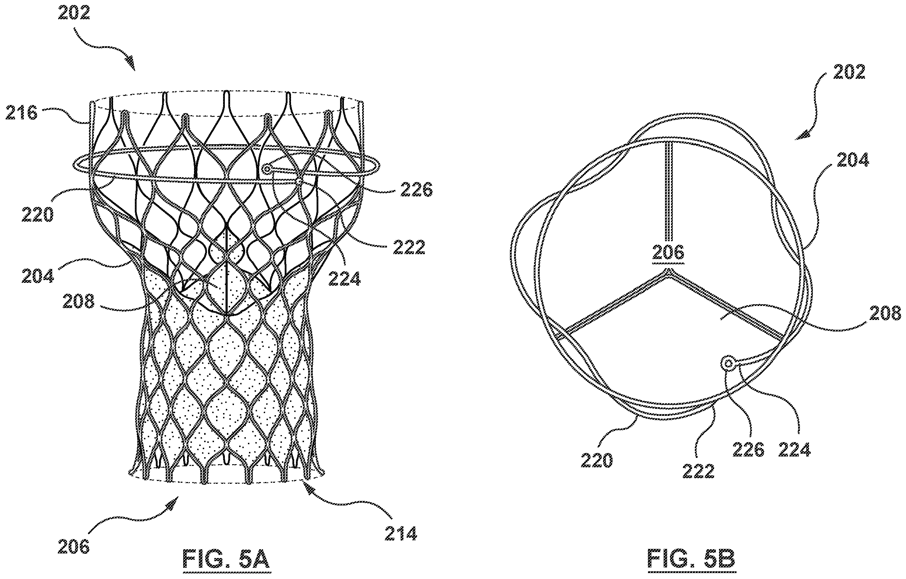

FIGS. 5A-7C illustrate schematically a valve assembly repositioning system 200 including a valve assembly 202 and a snare device 230 according to another embodiment hereof. Valve assembly repositioning system 200 is similar to valve assembly repositioning system 100 described above. In particular, valve assembly 202 is shown in FIGS. 5A-5B and includes a frame 204 and a prosthetic valve 208 as described above. Frame 204 and prosthetic valve 208 may be similar to frame 104 and prosthetic valve 108 describe above and the description in paragraphs [0040]-[0043] above, which are incorporated herein with respect to frame 204 and prosthetic valve 208. Thus, as described above, frame 204 includes a first end 214, a second end 216, and defines a central passage 206 therethrough. Frame 204 includes a number of wire members 210 arranged relative to each other to create open spaces 212. Prosthetic valve 208 is coupled to frame 204 and disposed within central passage of frame 204.

Similar to the embodiments described above, valve assembly further includes a repositioning wire 220. Repositioning wire 220 includes a first end 222 coupled to frame 204. Repositioning wire 220 wraps around a circumference of frame 204 to a second end 224, as shown in FIGS. 5A-5B. With valve assembly 202 in the radially expanded fully deployed configuration, repositioning wire 220 wraps around the circumference of frame 204 at least 75% of the circumference of frame 204 at the location of repositioning wire 220. As previously described, repositioning wire 220 may be woven through open spaces 212 of frame 204, above some frame members 210 and below others.

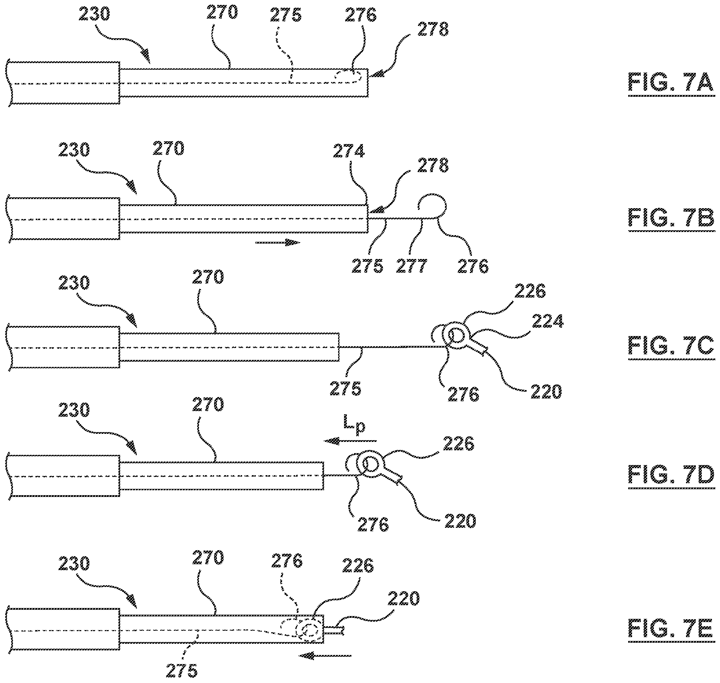

In the embodiment shown in of FIGS. 5A-7C, a lasso 226 is coupled to second end 224 of repositioning wire 220. Lasso 226 is of a generally circular shape creating a loop. Lasso 226 may be coupled to second end 224 by laser or ultrasonic welding, adhesives, or other methods suitable for the purposes disclosed herein. Alternatively, lasso 226 may be formed as an extension of repositioning wire 220 such that repositioning wire 220 forms a loop which is coupled to the remainder of repositioning wire 220 by a knot, laser or ultrasonic welding, adhesives, or other suitable connection methods. Repositioning wire 220 and lasso 226 may be made of the same materials described above for the repositioning wires.

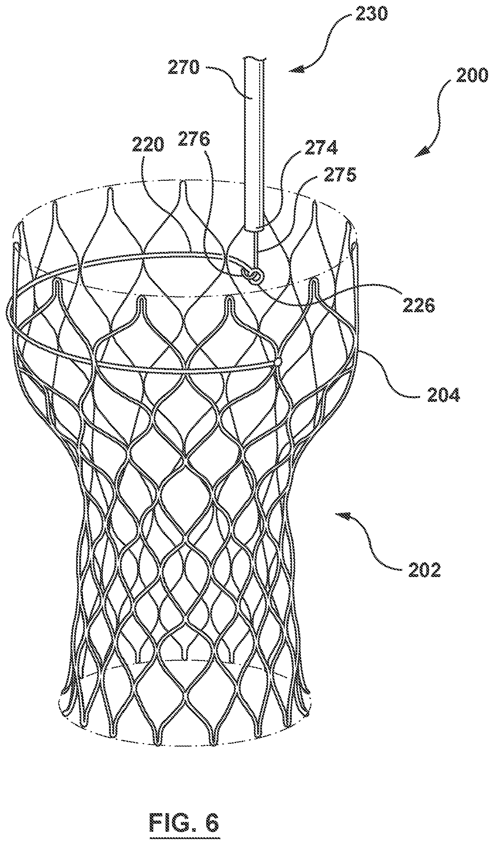

In the embodiment of FIGS. 5A-5B and 6, a single repositioning wire 220 is shown disposed around second end 216 of frame 204. However, as described above, more than one repositioning wire may be utilized. Further, repositioning wire 220 is not limited to the location at the second end 216 of frame 204.

FIGS. 7A-7E show an embodiment of snare device 230. Snare device 230 is an elongated device including a shaft 270 configured for delivery through the vasculature. A snare wire 275 including a snare mechanism 276 at a distal end 277 of snare wire 275 extends through a lumen 278 of shaft 270 and is slidable with respect to shaft 270. In an embodiment, snare mechanism 276 may be a hook, as shown, but other configurations suitable to snaring lasso 226 may also be used. In an embodiment, the position of snare mechanism 276 relative to shaft 270 is user selectable from a location at a proximal end (not shown) of snare device 23. Snare wire 275 and snare mechanism 276 may be constructed of materials such as, but not limited to stainless steel, Nitinol, nylon, polybutester, polypropylene, silk, and polyester or other materials suitable for the purposes described herein.

In an embodiment shown in FIGS. 7A-7E, snare device 230 may be delivered to a location of fully deployed valve assembly 202 with snare mechanism 276 disposed within lumen 278 of shaft 270, as shown in FIG. 7A. Snare device 230 may also be delivered with snare mechanism 276 distal of distal end 274 or snare wire may be delivered through lumen 278 after shaft 270 is delivered. When at the desired location, snare wire 275 is extended distally relative to shaft 270 such that snare mechanism 276 is distal of distal end 274 of shaft 270, as shown in FIG. 7B. Snare mechanism 276 is then manipulated to snare lasso 226 of repositioning wire 220, as shown in FIG. 6 and 7C. With snare mechanism 276 engaged with lasso 226, snare wire 275 may be retracted proximally in direction Lₚ to pull lasso 226 (and thus repositioning wire 220 coupled to lasso 226) towards shaft 270, as shown in FIG. 7D. As lasso 226 and repositioning wire 220 are pulled, valve assembly 202 is compressed from the radially expanded fully deployed configuration to the radially compressed repositioning configuration. Snare wire 275 may be retracted proximally until snare mechanism 276 and lasso 220 are disposed within lumen 278 of shaft 270, as shown in FIG. 7E.

With snare mechanism 276 engaged with lasso 226 and repositioning wire 220 pulled such that valve assembly 202 is in the radially compressed repositioning configuration, snare device 230 may be manipulated to move valve assembly 202 within the native vessel/valve. To move valve assembly 202 distally or proximally, the user pushes or pulls snare device 230, respectively. Stated another way, when snare device 230 has snared and pulled lasso 226 of repositioning wire 220, and valve assembly 202 is in the radially compressed repositioning configuration, moving snare device 230 distally moves valve assembly 202 distally, and moving snare device 230 proximally moves valve assembly 202 proximally.

As noted above, valve assembly 202 may include more than one repositioning wire. FIG. 8 shows valve assembly 202 including first and second repositioning wires 220A, 220B at first end 214 and second end 216 of frame 204, respectively. Valve assembly 202 shown in FIG. 8 is the same as the embodiment shown in FIGS. 5A-5B except for the additional repositioning wire. Thus, details of the valve assembly will not be repeated with respect to this embodiment, but the details described above with respect to other embodiments are incorporated herein.

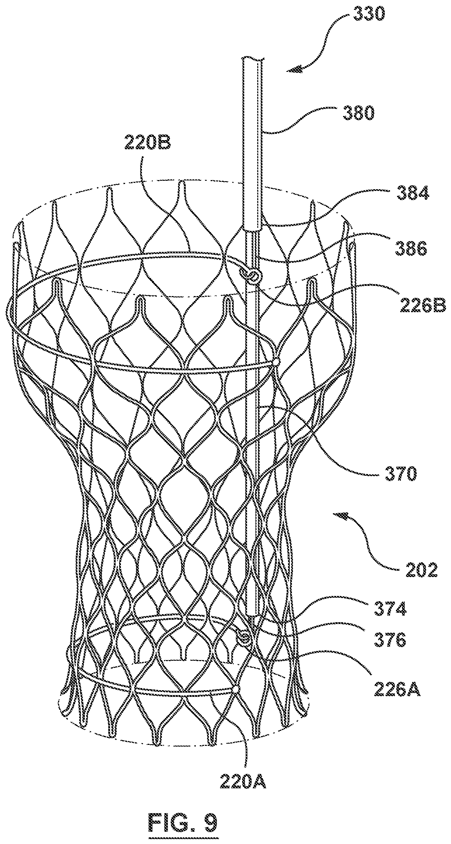

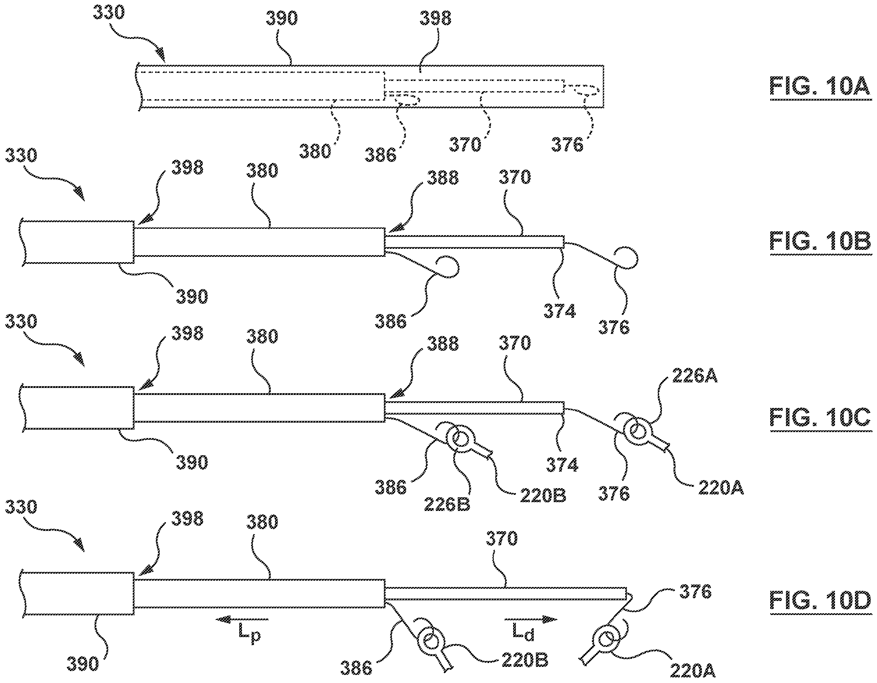

FIGS. 9 and 10A-10D show an embodiment of a snare device 330 which may be used in conjunction with valve assembly 202 including first and second repositioning wires 220A, 220B. Snare device 330 is an elongated device including a first snare 376 and a second snare 386. First snare 376 is disposed at a distal end 374 of a first shaft or wire 370. Second snare 386 is disposed at a distal end 384 of a second shaft 380. Second shaft 380 defines a lumen 388 through which first shaft 370 is slidably disposed. Snare device 330 may optionally include a third shaft 390 including a lumen 398. First and second shafts 370, 380 are slidable through third shaft 390. The position of first snare 376 relative to second snare 386 of snare device 330 is user adjustable by sliding first shaft 370 and second shaft 380 relative to each other. First snare 376 and second snare 386 are curved or sharply bent shapes suitable for catching, snagging, or snaring first lasso 226A and second lasso 226B, respectively. While FIGS. 9 and 10A-10D show first snare 376 and second snare 386 as a hook shape, this is not meant to limit the design and other shapes or constructions may be provided that are suitable for the purposes outlined herein. First and second snares 376, 386 may be constructed of materials such as, but not limited to stainless steel, Nitinol, nylon, polybutester, polypropylene, silk, and polyester or other materials suitable for the purposes described herein.

Snare device 330 may be advanced to a location of a fully deployed valve assembly in the configuration shown in FIG. 10A, with first and second shafts 370, 380 and first and second snares 376, 386 disposed within lumen 398 of third shaft 390. However, snare device 330 may be advanced to the location of a fully deployed valve assembly in other configurations. When at the desired location, first snare 376 and second snare 386 are exposed, for example, by retracting third shaft 390 or advancing first and second shafts 370, 380, as shown in FIG. 10B. First snare 376 and second snare 386 are manipulated such that first snare 376 is placed through first lasso 226A of repositioning wire 220A and second snare 386 is placed through second lasso 226B of repositioning wire 220B, as shown in FIG. 10C.

With first snare 376 engaged with first lasso 226A and second snare 386 engaged with second lasso 226B, first and second shafts 376, 386 may be moved in opposite directions, as indication by arrows L_{d} and Lₚ, respectively, in FIG. 10D. In the embodiment shown, first snare 376 is move distally and second snare 386 is moved proximally. Moving first snare 376 and second snare 386 in opposite directions causes first lasso 226A and second lasso 226B to be pulled, thereby causing first repositioning wire 220A and second repositioning wire 220B to be pulled, respectively. Pulling of first repositioning wire 220A and second repositioning wire 220B radially compresses valve assembly 202 from the radially expanded fully deployed configuration to the radially compressed repositioning configuration, as explained above.

With first and second snares 376, 386 engaged with first and second lassos 226A, 226B and first and second repositioning wires 220A, 220B pulled such that valve assembly 202 is in the radially compressed repositioning configuration, snare device 330 may be manipulated to move valve assembly 202 within the native vessel/valve. To move valve assembly 202 distally or proximally within the native valve, the user moves snare device 330 distally or proximally, respectively. Stated another way, when snare device 330 has snared and pulled first and second lassos 226A, 226B of first and second repositioning wires 220A, 220B, respectively, and valve assembly 202 is in the radially compressed repositioning configuration, moving snare device 330 distally moves valve assembly 202 distally, and moving snare device 330 proximally moves valve assembly 202 proximally.

An example, not forming part of the invention, of a method of repositioning a fully deployed valve assembly in a native valve is schematically represented in FIGS. 11-16. Although the method is described with respect to valve assembly 102 and snare device 130, it will be apparent to one of ordinary skill that methods described herein may be utilized with valve assemblies and snare devices according to any embodiment described herein. In FIG. 11, a valve assembly 102 is fully deployed adjacent a native valve 700 at a first location. Valve assembly 102 is in a radially expanded fully deployed configuration and is thus disconnected from a delivery device. As previously described, valve assembly 102 includes a frame 104, a repositioning wire 120, and a prosthetic valve 108. As determined by the treating clinician, valve assembly 102 may not be performing as desired, and repositioning of valve assembly 102 is desired to improve valve performance. For example, and not by way of limitation, it may be determined that valve assembly 102 is "too deep" into the annulus 702 such that it may interfere with the left ventricular outflow tract (LVOT) or form gaps between frame 104 and annulus 702 causing paravalvular leakage. In another example, valve assembly 102 may not be deep enough in annulus such that frame 104 is not properly secured against annulus 702, which may also cause paravalvular leakage.

Snare device 130 is advanced through the patient's vasculature and is positioned adjacent valve assembly 102. Clasping mechanism 176 of snare device 130 is manipulated by the treating clinician to grasp a second end 124 of repositioning wire 120, as shown in FIG. 12.

Snare device 130 is rotated in a direction R1 by the treating clinician, thereby pulling repositioning wire 120 as it wraps circumferentially around shaft 170 of snare device 130. The pulling of repositioning wire 120 compresses valve assembly 102 from the radially expanded fully deployed configuration to the radially compressed repositioning configuration, as shown in FIG. 13.

Snare device 130 may then be moved proximally or distally to reposition valve assembly 102 from the first location of FIG. 11 to a second location adjacent native valve 700. FIG. 14 shows snare device 130 and valve assembly 102 being moved proximally in a direction Lₚ to the second location as determined by the treating clinician. However, valve assembly 102 may be moved distally.

Snare device 130 is rotated in a direction R2 opposite direction R1 by the treating clinician, thereby releasing the pulling force on repositioning wire 120 as repositioning wire 120 unwraps circumferentially from shaft 170 of snare device 130. As the pulling force is released, frame 104 of valve assembly 102 self-expands from the radially compressed repositioning configuration to the radially expanded fully deployed configuration at the second location, as shown in FIG. 15.

Once valve assembly 102 is in its radially expanded fully deployed configuration at the second location, snare device 130 may be withdrawn from the patient. Valve assembly 102 remains fully deployed at the repositioned second location adjacent native valve 700, as shown in FIG. 16.

A similar method, not forming part of the invention, may be used for a valve assembly 102 including a plurality of repositioning wires 120. For example, and not by way of limitation, a snare device may be used for each repositioning wire. In another example, a snare device may include multiple clasping mechanisms, one for each of the plurality of repositioning wires. Further, a similar method may be used to reposition valve assembly 202 of FIGS. 5A-5B utilizing the snare device of FIGS. 7A-7E.

FIGS. 17-22 schematically show a method, not forming part of the invention, of repositioning a fully deployed valve assembly 202. Although described herein with respect to valve assembly 202 of FIGS. 8A-8B and snare device 330 of FIGS. 10A-10D, it will be apparent to one of ordinary skill that methods described herein may be used with valve assemblies and snare devices according to any embodiment described herein. In FIG. 17, a valve assembly 202 is fully deployed adjacent a native valve 700 at a first location. Valve assembly 202 is in a radially expanded fully deployed configuration and thus is disconnected from a delivery device. As previously described, valve assembly 202 includes a frame 204, first and second repositioning wires 220A, 220B, and a prosthetic valve 208. As determined by the treating clinician, valve assembly 202 may not be performing as desired, and repositioning of valve assembly 202 is desired to improve valve performance. For example, and not by way of limitation, it may be determined that valve assembly 202 is "too deep" into the annulus 702 such that it may interfere with the left ventricular outflow tract (LVOT) or form gaps between frame 204 and annulus 702 causing paravalvular leakage. In another example, valve assembly 202 may not be deep enough in annulus such that frame 204 is not properly secured against annulus 702, which may also cause paravalvular leakage.

Snare device 330 is advanced through the patient's and is positioned within valve assembly 202, as shown in FIG. 18.

First snare 376 is manipulated by the treating clinician to snare first lasso 226A of first repositioning wire 220A and second snare 386 is manipulated by the treating clinician to snare second lasso 226B of second repositioning wire 220B, as shown in FIG. 18. Once first lasso 226A and second lasso 226B are snared by first snare 376 and second snare 386, respectively, snare device 330 is actuated by the treating clinician such that first snare 376 and second snare 386 are moved apart from each other. This movement causes first repositioning wire 220A and second repositioning wire 220B to be pulled in directions L_{d} and Lₚ, respectively, thereby compressing valve assembly 202 from the radially expanded fully deployed configuration to a radially compressed repositioning configuration, as shown in FIG. 19.

Snare device 330 may then be moved proximally or distally to reposition valve assembly 202 from the first location of FIG. 17 to a second location adjacent the native valve 700. FIG. 20 shows snare device 330 and valve assembly 202 being moved proximally in a direction Lₚ to the second location as determined by the treating clinician. However, valve assembly 202 may instead be moved distally.

When valve assembly 202 is positioned at the repositioned second location, the treating clinician may move first snare 376 and second snare 386 back toward each other such that the pulling force on first repositioning wire 220A and second repositioning wire 220B is released. As the pulling force on repositioning wires 220A and 220B is released, valve assembly 202 self-expands from the radially compressed repositioning configuration to the radially expanded fully deployed configuration at the second location, as shown in FIG. 21.

Once valve assembly 202 is and in its radially expanded fully deployed configuration at the second location, snare device 330 may be withdrawn from the patient. Valve assembly 202 remains fully deployed at the repositioned second location adjacent native valve 700, as shown in FIG. 22.

While only some embodiments and methods, the methods not forming part of the invention, have been described herein, it should be understood that it has been presented by way of illustration and example only, and not limitation. Various changes in form and detail can be made therein without departing from the scope of the invention, which is defined by the appended claims.

## Claims

1. A valve assembly (102, 202) having a radially expanded fully deployed configuration, a radially compressed delivery configuration, and a radially compressed repositioning configuration, the valve assembly (102, 202) comprising:
a generally tubular frame (104, 204) defining a central passage (106, 206);
a prosthetic valve (108, 208) coupled to the frame (104, 204) and disposed in the central passage (106, 206); and
a repositioning wire (120, 220) coupled to the frame (104, 204) and configured such that pulling the repositioning wire (120, 220) radially compresses the valve assembly (102, 202) from the radially expanded fully deployed configuration to the radially compressed repositioning configuration,
wherein the radially compressed delivery configuration is a smaller diameter than the radially compressed repositioning configuration.

2. The valve assembly (102, 202) of claim 1, wherein the frame (104, 204) includes a plurality of frame members (110, 210) with open spaces (112, 212) between the frame members (110, 210), wherein the repositioning wire (120, 220) is woven through the open spaces (112, 212), above some frame members (110, 210), and below other frame members (110, 210).

3. The valve assembly (102, 202) of claim 1, wherein the repositioning wire (120, 220) comprises a plurality of repositioning wires (120A, 120B, 220A, 220B).

4. The valve assembly (102, 202) of claim 1, wherein the repositioning wire (120, 220) has a first end (122A, 122B, 222) coupled to the frame (104, 204), wherein the repositioning wire (120, 220) extends from the first end (122A, 122B, 222) around at least a portion of a circumference of the frame (104, 204) to a second end (124, 224) of the repositioning wire (120, 220).

5. The valve assembly (102, 202) of claim 4, wherein with the valve assembly (102, 202) in the radially expanded fully deployed configuration, the repositioning wire (120, 220) extends around at least 75 percent of the circumference of the frame (104, 204).

6. The valve assembly (202) of claim 4, wherein the second end (224) of the repositioning wire (220) includes a lasso (226).

7. The valve assembly (102, 202) of claim 1, wherein a diameter (D_{c}) of the frame (104, 204) in the radially compressed repositioning configuration is in the range of 40-80 percent of a diameter (Dₑ) of the frame (104, 204) in the radially expanded fully deployed configuration.

8. A valve assembly repositioning system comprising:
a valve assembly (102, 202) according to claim 1; and
a snare device (130, 230, 330) configured to snare the repositioning wire (120, 220) and to pull the repositioning wire (120, 220) to radially compress the valve assembly (102, 202) from the radially expanded fully deployed configuration to the radially compressed repositioning configuration, wherein the snare device (130, 230, 330) is further configured to move the valve assembly (102, 202) when the valve assembly (102, 202) is in the radially compressed repositioning configuration.

9. The valve assembly repositioning system of claim 8, wherein the repositioning wire (120, 220) comprises a plurality of repositioning wires (120A, 120B, 220A, 220B).

10. The valve assembly repositioning system of claim 8, wherein the repositioning wire (120, 220) has a first end (122A, 122B, 222) coupled to the frame (104, 204), wherein the repositioning wire (120, 220) extends from the first end (122A, 122B, 222) around at least a portion of a circumference of the frame (104, 204) to a second end (124, 224) of the repositioning wire (120, 220).

11. The valve assembly of claim 10, wherein with the valve assembly (102, 202) in the radially expanded fully deployed configuration, the repositioning wire (120, 220) extends around at least 75 percent of the circumference of the frame (104, 204).

12. The valve assembly repositioning system of claim 8, wherein the snare device (130) includes a clasping mechanism (176) at a distal second end (174) of the snare device (130).

13. The valve assembly repositioning system of claim 8, wherein the repositioning wire (220) includes a lasso (226) or
wherein a diameter (D_{c}) of the frame (104, 204) in the radially compressed repositioning configuration is in the range of 40-80 percent of a diameter (Dₑ) of the frame (104, 204) in the radially expanded fully deployed configuration.

14. The valve assembly repositioning system of claim 8,
wherein the repositioning wire (220) includes a first repositioning wire (220A) disposed adjacent a first end (214) of the frame (204) and including a first lasso (226A), and a second repositioning wire (220B) disposed adjacent a second end (216) of the frame (204) and including a second lasso (226B),
wherein the snare device (330) includes a first snare (376) configured to snare the first lasso (226A) and a second snare (386) configured to snare the second lasso (226B), and
wherein the snare device (330) is configured such that the first snare (376) and the second snare (386) are moved apart from each other to move the first lasso (226A) and the second lasso (226B) apart from each other to radially compress the valve assembly (202).

15. The valve assembly repositioning system of claim 14, wherein the first snare (376) is disposed at a distal end (374) of a first shaft (370) and the second snare (386) is disposed at a distal end (384) of a second shaft (380), wherein the first shaft (370) is disposed within a lumen (388) of and is slidable relative to the second shaft (380).

## Patentansprüche

1. Klappenanordnung (102, 202), die eine radial expandierte, vollständig entfaltete Konfiguration, eine radial komprimierte Abgabekonfiguration und eine radial komprimierte Neupositionierungskonfiguration aufweist, die Klappenanordnung (102, 202) umfassend:
einen im Allgemeinen rohrförmigen Rahmen (104, 204), der einen zentralen Durchgang (106, 206) definiert;
eine prothetische Klappe (108, 208), die mit dem Rahmen (104, 204) gekoppelt und in dem zentralen Durchgang (106, 206) angeordnet ist; und
einen Neupositionierungsdraht (120, 220), der mit dem Rahmen (104, 204) gekoppelt und derart konfiguriert ist, dass ein Ziehen des Neupositionierungsdrahts (120, 220) die Klappenanordnung (102, 202) von der radial expandierten, vollständig entfalteten Konfiguration zu der radial komprimierten Neupositionierungskonfiguration radial komprimiert,
wobei die radial komprimierte Abgabekonfiguration einen kleineren Durchmesser als die radial komprimierte Neupositionierungskonfiguration ist.

2. Klappenanordnung (102, 202) nach Anspruch 1, wobei der Rahmen (104, 204) eine Vielzahl von Rahmenelementen (110, 210) mit offenen Räumen (112, 212) zwischen den Rahmenelementen (110, 210) beinhaltet, wobei der Neupositionierungsdraht (120, 220) durch die offenen Räume (112, 212) über einige Rahmenelemente (110, 210) und unter anderen Rahmenelementen (110, 210) gewebt wird.

3. Klappenanordnung (102, 202) nach Anspruch 1, wobei der Neupositionierungsdraht (120, 220) eine Vielzahl von Neupositionierungsdrähten (120A, 120B, 220A, 220B) umfasst.

4. Klappenanordnung (102, 202) nach Anspruch 1, wobei der Neupositionierungsdraht (120, 220) ein erstes Ende (122A, 122B, 222) aufweist, das mit dem Rahmen (104, 204) gekoppelt ist, wobei der Neupositionierungsdraht (120, 220) sich von dem ersten Ende (122A, 122B, 222) um mindestens einen Abschnitt eines Umfangs des Rahmens (104, 204) herum zu einem zweiten Ende (124, 224) des Neupositionierungsdrahts (120, 220) erstreckt.

5. Klappenanordnung (102, 202) nach Anspruch 4, wobei, wenn sich die Klappenanordnung (102, 202) in der radial expandierten, vollständig entfalteten Konfiguration befindet, der Neupositionierungsdraht (120, 220) sich um mindestens 75 Prozent des Umfangs des Rahmens (104, 204) herum erstreckt.

6. Klappenanordnung (202) nach Anspruch 4, wobei das zweite Ende (224) des Neupositionierungsdrahts (220) ein Lasso (226) beinhaltet.

7. Klappenanordnung (102, 202) nach Anspruch 1, wobei ein Durchmesser (D_{c}) des Rahmens (104, 204) in der radial komprimierten Neupositionierungskonfiguration in dem Bereich von 40 bis 80 Prozent eines Durchmessers (Dₑ) des Rahmen (104, 204) in der radial expandierten, vollständig entfalteten Konfiguration liegt.

8. Neupositionierungssystem für eine Klappenanordnung, umfassend:
eine Klappenanordnung (102, 202) nach Anspruch 1; und
eine Schlingenvorrichtung (130, 230, 330), die konfiguriert ist, um den Neupositionierungsdraht (120, 220) zu umschlingen und den Neupositionierungsdraht (120, 220) zu ziehen, um die Klappenanordnung (102, 202) aus der radial expandierten, vollständig entfalteten Konfiguration zu der radial komprimierten Neupositionierungskonfiguration radial zu komprimieren, wobei die Schlingenvorrichtung (130, 230, 330) ferner konfiguriert ist, um die Klappenanordnung (102, 202) zu bewegen, wenn sich die Klappenanordnung (102, 202) in der radial komprimierten Neupositionierungskonfiguration befindet.

9. Neupositionierungssystem für die Klappenanordnung nach Anspruch 8, wobei der Neupositionierungsdraht (120, 220) eine Vielzahl von Neupositionierungsdrähten (120A, 120B, 220A, 220B) umfasst.

10. Neupositionierungssystem für die Klappenanordnung nach Anspruch 8, wobei der Neupositionierungsdraht (120, 220) ein erstes Ende (122A, 122B, 222) aufweist, das mit dem Rahmen (104, 204) gekoppelt ist, wobei sich der Neupositionierungsdraht (120, 220) von dem ersten Ende (122A, 122B, 222) um mindestens einen Abschnitt eines Umfangs des Rahmens (104, 204) bis zu einem zweiten Ende (124, 224) des Neupositionierungsdrahts (120, 220) herum erstreckt.

11. Klappenanordnung nach Anspruch 10, wobei, wenn sich die Klappenanordnung (102, 202) in der radial expandierten, vollständig entfalteten Konfiguration befindet, der Neupositionierungsdraht (120, 220) sich um mindestens 75 Prozent des Umfangs des Rahmens (104, 204) herum erstreckt.

12. Neupositionierungssystem für die Klappenanordnung nach Anspruch 8, wobei die Schlingenvorrichtung (130) einen Klammermechanismus (176) an einem distalen zweiten Ende (174) der Schlingenvorrichtung (130) beinhaltet.

13. Neupositionierungssystem für die Klappenanordnung nach Anspruch 8, wobei der Neupositionierungsdraht (220) ein Lasso (226) beinhaltet oder
wobei ein Durchmesser (D_{c}) des Rahmens (104, 204) in der radial komprimierten Neupositionierungskonfiguration in dem Bereich von 40 bis 80 Prozent eines Durchmessers (Dₑ) des Rahmens (104, 204) in der radial expandierten, vollständig entfalteten Konfiguration liegt.

14. Neupositionierungssystem für die Klappenanordnung nach Anspruch 8,
wobei der Neupositionierungsdraht (220) einen ersten Neupositionierungsdraht (220A), der angrenzend an ein erstes Ende (214) des Rahmens (204) angeordnet ist und ein erstes Lasso (226A) beinhaltet, und einen zweiten Neupositionierungsdraht (220B) beinhaltet, der angrenzend an ein zweites Ende (216) des Rahmens (204) angeordnet ist und ein zweites Lasso (226B) beinhaltet,
wobei die Schlingenvorrichtung (330) eine erste Schlinge (376), die konfiguriert ist, um das erste Lasso (226A) zu schlingen, und eine zweite Schlinge (386) beinhaltet, die konfiguriert ist, um das zweite Lasso (226B) zu schlingen, und
wobei die Schlingenvorrichtung (330) derart konfiguriert ist, dass die erste Schlinge (376) und die zweite Schlinge (386) voneinander weg bewegt werden, um das erste Lasso (226A) und das zweite Lasso (226B) voneinander weg zu bewegen, um die Klappenanordnung (202) radial zu komprimieren.

15. Neupositionierungssystem für die Klappenanordnung nach Anspruch 14, wobei die erste Schlinge (376) an einem distalen Ende (374) eines ersten Schafts (370) angeordnet ist und die zweite Schlinge (386) an einem distalen Ende (384) des zweiten Schafts (380) angeordnet ist, wobei der erste Schaft (370) innerhalb eines Lumens (388) des zweiten Schafts (380) angeordnet und relativ zu diesem verschiebbar ist.

## Revendications

1. Ensemble valve (102, 202) ayant une configuration entièrement déployée radialement expansée, une configuration de distribution radialement comprimée et une configuration de repositionnement radialement comprimée, l'ensemble valve (102, 202) comprenant :
un cadre généralement tubulaire (104, 204) définissant un passage central (106, 206) ;
une valve prothétique (108, 208) accouplée au cadre (104, 204) et disposée dans le passage central (106, 206) ; et
un fil de repositionnement (120, 220) accouplé au cadre (104, 204) et conçu de telle sorte que la traction du fil de repositionnement (120, 220) comprime radialement l'ensemble valve (102, 202) de la configuration entièrement déployée radialement expansée à la configuration de repositionnement radialement comprimée,
la configuration de distribution radialement comprimée étant d'un diamètre plus petit que la configuration de repositionnement radialement comprimée.

2. Ensemble valve (102, 202) selon la revendication 1, dans lequel le cadre (104, 204) comporte une pluralité d'éléments de cadre (110, 210) avec des espaces ouverts (112, 212) entre les éléments de cadre (110, 210), le fil de repositionnement (120, 220) étant tissé à travers les espaces ouverts (112, 212), au-dessus de certains éléments de cadre (110, 210) et en dessous d'autres éléments de cadre (110, 210).

3. Ensemble valve (102, 202) selon la revendication 1, dans lequel le fil de repositionnement (120, 220) comprend une pluralité de fils de repositionnement (120A, 120B, 220A, 220B).

4. Ensemble valve (102, 202) selon la revendication 1, dans lequel le fil de repositionnement (120, 220) a une première extrémité (122A, 122B, 222) accouplée au cadre (104, 204), le fil de repositionnement (120, 220) s'étendant de la première extrémité (122A, 122B, 222) autour d'au moins une partie d'une circonférence du cadre (104, 204) jusqu'à une seconde extrémité (124, 224) du fil de repositionnement (120, 220).

5. Ensemble valve (102, 202) selon la revendication 4, dans lequel, avec l'ensemble valve (102, 202) dans la configuration entièrement déployée radialement expansée, le fil de repositionnement (120, 220) s'étend autour d'au moins 75 % de la circonférence du cadre (104, 204).

6. Ensemble valve (202) selon la revendication 4, dans lequel la seconde extrémité (224) du fil de repositionnement (220) comporte un lasso (226).

7. Ensemble valve (102, 202) selon la revendication 1, dans lequel un diamètre (D_{c}) du cadre (104, 204) dans la configuration de repositionnement radialement comprimée est compris dans la plage de 40 à 80 % d'un diamètre (Dₑ) du cadre (104, 204) dans la configuration entièrement déployée radialement expansée.

8. Système de repositionnement d'ensemble valve comprenant :
un ensemble valve (102, 202) selon la revendication 1 ; et
un dispositif d'anse (130, 230, 330) conçu pour accrocher le fil de repositionnement (120, 220) et pour tirer le fil de repositionnement (120, 220) afin de comprimer radialement l'ensemble valve (102, 202) à partir de la configuration entièrement déployée radialement expansée à la configuration de repositionnement radialement comprimée, le dispositif d'anse (130, 230, 330) étant en outre conçu pour déplacer l'ensemble valve (102, 202) lorsque l'ensemble valve (102, 202) est dans la configuration de repositionnement radialement comprimée.

9. Système de repositionnement d'ensemble valve selon la revendication 8, dans lequel le fil de repositionnement (120, 220) comprend une pluralité de fils de repositionnement (120A, 120B, 220A, 220B).

10. Système de repositionnement d'ensemble valve selon la revendication 8, dans lequel le fil de repositionnement (120, 220) a une première extrémité (122A, 122B, 222) accouplée au cadre (104, 204), le fil de repositionnement (120, 220) s'étendant à partir de la première extrémité (122A, 122B, 222) autour d'au moins une partie d'une circonférence du cadre (104, 204) jusqu'à une seconde extrémité (124, 224) du fil de repositionnement (120, 220).

11. Ensemble valve selon la revendication 10, dans lequel, avec l'ensemble valve (102, 202) dans la configuration entièrement déployée radialement expansée, le fil de repositionnement (120, 220) s'étend autour d'au moins 75 pour cent de la circonférence du cadre (104, 204).

12. Système de repositionnement d'ensemble valve selon la revendication 8, dans lequel le dispositif d'anse (130) comporte un mécanisme de serrage (176) à une seconde extrémité distale (174) du dispositif d'anse (130).

13. Système de repositionnement d'ensemble valve selon la revendication 8, dans lequel le fil de repositionnement (220) comporte un lasso (226) ou
un diamètre (D_{c}) du cadre (104, 204) dans la configuration de repositionnement radialement comprimée étant compris dans la plage de 40 à 80 pour cent d'un diamètre (Dₑ) du cadre (104, 204) dans la configuration entièrement déployée radialement expansée.

14. Système de repositionnement d'ensemble valve selon la revendication 8,
le fil de repositionnement (220) comportant un premier fil de repositionnement (220A) disposé de manière adjacente à une première extrémité (214) du cadre (204) et comportant un premier lasso (226A), et un second fil de repositionnement (220B) disposé de manière adjacente à une seconde extrémité (216) du cadre (204) et comportant un second lasso (226B),
le dispositif d'anse (330) comportant une première anse (376) conçue pour accrocher le premier lasso (226A) et une seconde anse (386) conçue pour accrocher le second lasso (226B), et
le dispositif d'anse (330) étant conçu de telle sorte que la première anse (376) et la seconde anse (386) sont éloignées l'une de l'autre pour éloigner le premier lasso (226A) et le second lasso (226B) l'un de l'autre pour comprimer radialement l'ensemble valve (202).

15. Système de repositionnement d'ensemble valve selon la revendication 14, dans lequel la première anse (376) est disposée à une extrémité distale (374) d'un premier arbre (370) et la seconde anse (386) est disposée à une extrémité distale (384) d'un second arbre (380), le premier arbre (370) étant disposé à l'intérieur d'une lumière (388) du second arbre (380) et pouvant coulisser par rapport à celui-ci.
